# EUROPEAN PATENT APPLICATION

(11) **EP 2 527 003 A1**
(43) Date of publication of application: **28.11.2012**
(21) Application number: 12004071.2
(22) Date of filing: 25.05.2012
(51) Int. Cl.: A61N 1/44, A61N 7/00, A61M 37/00

(54) **Apparatus for the anti-inflammatory treatment of osteoarthritis, spinal column, traumas, joint inflammations as well as of the entire musculoskeletal system**

(30) Priority: 26.05.2011 IT PS20110008
(71) Applicant: Ponselè, Michele, 61122 Pesaro PU (IT); Barboni, Antonio, 61030 Cartoceto PU (IT); Ivaldi, Cesare, 60128 Ancona (AN) (IT); Romiti, Morena, 61024 Mombaroccio PU (IT)
(72) Inventor: Ponselè, Michele, 61122 Pesaro PU (IT)
(74) Representative: Cacciamani, Clizia

(57) **Abstract**

Apparatus for the anti-inflammatory treatment of osteoarthritis, spinal column, traumas, joint Inflammations as well as of the entire musculoskeletal system, comprising electronic circuits and an ionization chamber with at least one electrode; said apparatus comprises a radio frequency generator (1) modulated by a low frequency modulator (3) and connected to a power amplifier (4), said circuit feeding a transducer (5) comprising at least one electrode (8) and a tubular ionisation chamber (9) terminating with an open end (10) which is positioned, during treatment, at the site to be treated at a suitable distance from the skin.

## Description

### Text of the description

The currently available technology involves the non-concurrent use of the ozone-based therapy, the therapy with sonic and ultrasonic waves, and the therapy with high-frequency electric fields.

These treatments are carried out with different machines using systems very different In nature for the application of the treatment to the patient.

Our apparatus is a revolutionary machine for the anti-inflammatory treatment of osteoarthritis, spinal column, traumas, joint inflammations as well as of the entire musculoskeletal system. With the present machine, we were able to combine and merge those three technologies, thus achieving outstanding results In the anti-inflammatory field.

The accompanying tables of drawings show an example of the apparatus.
Figure 1 is a block diagram of the device which generates the desired frequency;
Figure 2 is a block diagram of the transducer;
Figure 3 is a detail of the ionization chamber; and
Figure 4 is a plan view of the ionization chamber.

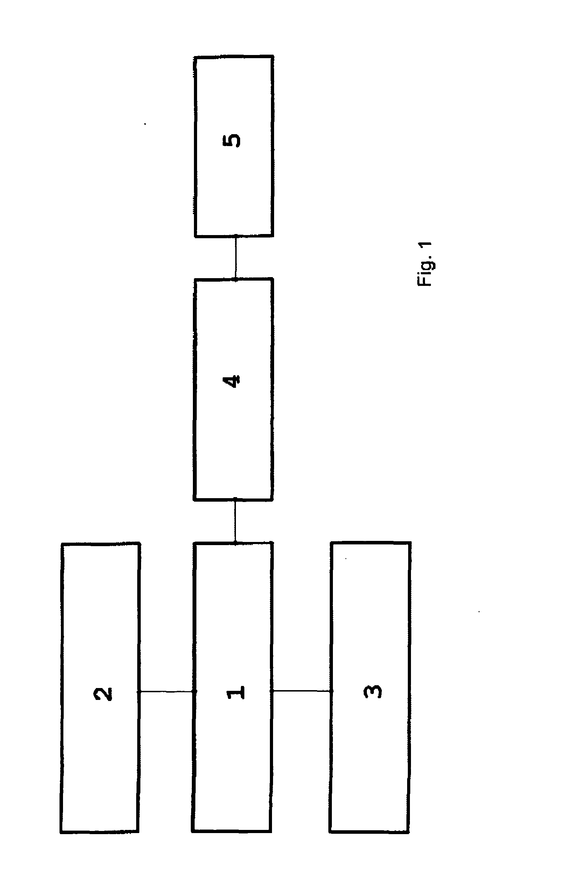

As can be seen from Figure 1, the apparatus comprises a radio frequency generator 1 operating at a frequency in the range from 300 kHz to 450 kHz and capable of being optimally tuned by means of a fine adjusting circuit 2 which allows the system to deliver the maximum power according to the application being considered, and furthermore there is a modulating module 3 which allows the high frequency to be supplemented with a modulating feature which is extremely important for the final result. The modulating signal Is a square wave with a frequency of about 1 KHz.

In addition, there is a power module 4 which appropriately amplifies the starting signal and makes it usable for the transducer 5, which Is the member intended for applying our therapy to the patient.

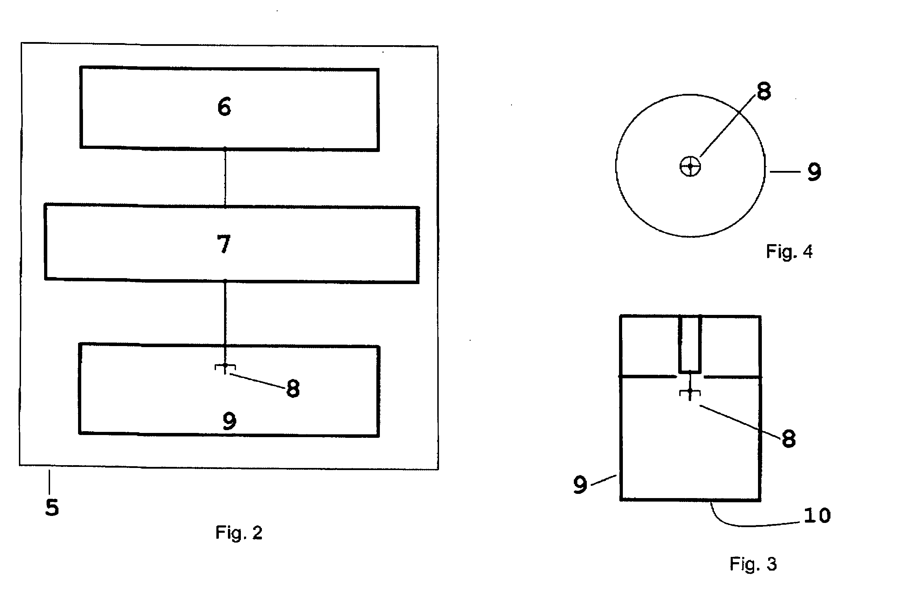

Figure 2 shows the construction of the transducer, which can have various shapes and sizes depending on the purposes for which it is employed but which has, however, the parts shown In the drawings of Figures 2 and 3 as the basic parts for the correct operation thereof.

As a first member, there is a circuit 6 tuned to the typical frequency of the RF generator, said tuned circuit providing its energy to a voltage-increasing coll system 7 which increases the voltage at the ends of an electrode 8 to above 20 kV. This electrode of special shape and material is placed in a chamber referred to as an ionization/sonic compression chamber 9. The synthesis of the three principles occurs within this camber. The air existing In the chamber is enriched with ozone (O₃) which is generated in the same chamber by means of a tip to which a very high voltage is applied. The modulation applied to the radio frequency carrier makes the walls of the chamber to oscillate so as to create pressure waves compressing the mixture of air and ozone. The ionization chamber 9 of tubular shape is positioned with the open end portion 10 at a distance from the skin of between 2.5 cm and 0.5 cm, and preferably of about 1,0 cm, at the site where the treatment is to be carried out. The high frequency generated in the chamber ionizes the environment so as to open channels - referred to as transdermal channels, which can extend up to several centimetres in depth - through the skin and the underlying tissue. These channels allow the mixture of air and ozone pressed and promoted by the sonic waves to penetrate deeply and release the beneficial anti-inflammatory effect typical of ozone. When the ionizing effect of the high frequency is ceased, these channels tend to close, thus leaving the ozone trapped in depth and enabling it to slowly provide its oxygenating power to the affected tissues.

In a possible variant embodiment of the device, the tuning circuit 6 and the voltage increasing circuit 7 can be incorporated into the main generator, so that the ionization chamber 9 only contains the electrode 8. Although the present apparatus is particularly effective for the anti-inflammatory treatment of osteoarthritis, spinal column, traumas, joint Inflammations as well as of the whole musculoskeletal system, it could also be used for other treatments in which it is required to carry drugs or products even intended for an aesthetic use, if needed. In fact, due to the ionization field generated by the apparatus, the apparatus is suitable for carrying products which are either directly applied to the skin or atomized and filled into the ionization chamber, in a non-invasive manner.

From the above description, it can be seen that this system can simply and effectively combine the three physical principles required to obtain a machine which can make a medicament, a drug or any other substance to penetrate deeply and painlessly without using annoying needles or electric discharges, In a painless and non-invasive manner.

With the use of this system, the only effect perceived by the patient is a beneficial heat on the part under treatment when the treatment is being applied, and thereafter a partial reduction of pain which tends to be completely abolished after a few sessions, as determined by the protocol being used.

## Claims

1. Apparatus for the anti-inflammatory treatment of osteoarthritis, spinal column, traumas, joint inflammations as well as of the entire musculoskeletal system, comprising electronic circuits and an ionization chamber with at least one electrode, **characterized in that** it comprises a radio frequency generator (1) modulated by a low frequency modulator (3) and connected to a power amplifier (4), said circuit feeding a transducer (5) comprising at least one electrode (8) and a tubular ionisation chamber (9) terminating with an open end (10) which is positioned, during treatment, at the site to be treated at a suitable distance from the skin.

2. Apparatus according to claim 1, **characterized in that** the generator (1) can be adjusted to the optimum frequency by means of a manual or automatic adjusting circuit (2).

3. Apparatus according to claim 1 or claim 2, **characterized In that** the modulation applied to the radio frequency carrier makes the walls of the chamber to oscillate so as to create pressure waves which compress the mixture of air and ozone.

4. Apparatus according to any one of the preceding claims 1 to 3, **characterized In that** said transducer (5) comprises a high-frequency tuning circuit (6) and a voltage increasing circuit (7) which feeds an electrode (8) located in an ionization/sonic compression chamber (9).

5. Apparatus according to any one of the preceding claims 1 to 4, **characterized In that** the ionization chamber (9) with the respective electrode (8) is separated from the high-frequency tuning circuit (6) and the voltage increasing circuit (7) which are Incorporated into the main generator.

6. Apparatus according to any one of the preceding claims 1 to 5, wherein said end (10) of the ionisation chamber (9) is positioned, during treatment, at a distance from the skin of from about 2,5 cm to 0.5 cm.
